# EUROPEAN PATENT SPECIFICATION

(11) **EP 3 675 725 B1**
(45) Date of publication and mention of the grant of the patent: **28.09.2022**
(21) Application number: 17811731.3
(22) Date of filing: 28.08.2017
(51) Int. Cl.: A61B 5/389, A61B 5/397, A61B 5/11, G16H 50/20

(54) **AN IMPROVED DEVICE FOR DETECTING, PREVENTING, MONITORING AND TREATING PARAFUNCTIONAL ACTIVITIES IN ODONTOLOGICAL FIELD**
VERBESSERTE VORRICHTUNG ZUR ERKENNUNG, VORBEUGUNG, ÜBERWACHUNG UND BEHANDLUNG VON PARAFUNKTIONELLEN AKTIVITÄTEN IN DER ZAHNHEILKUNDE
DISPOSITIF AMÉLIORÉ POUR DÉTECTER, PRÉVENIR, SURVEILLER ET TRAITER LES ACTIVITÉS PARAFONCTIONNELLES DANS LE DOMAINE ODONTOLOGIQUE

(43) Date of publication of application: 08.07.2020
(73) Proprietor: Biotechnovations S.r.l. con unico socio, 18038 Sanremo (IM) (IT)
(72) Inventor: NOVERI, Lorenzo, 18038 Sanremo (IM) (IT)
(74) Representative: Emmi, Mario
(86) International application number: PCT/IT2017/000182
(87) International publication number: WO 2019/043736

(56) References cited:
- DE-U1-202009 012 470
- US-A1- 2006 184 059
- M. FUJISAWA ET AL: "Determination of daytime clenching events in subjects with and without self-reported clenching", JOURNAL OF ORAL REHABILITATION, vol. 40, 1 August 2013 (2013-08-01), pages 731-736, XP55476978, GB ISSN: 0305-182X, DOI: 10.1111/joor.12087
- AKIRA WATANABE ET AL: "Effect of electromyogram biofeedback on daytime clenching behavior in subjects with masticatory muscle pain", JOURNAL OF PROSTHODONTIC RESEARCH, ELSEVIER, AMSTERDAM, NL, vol. 55, no. 2, 20 September 2010 (2010-09-20), pages 75-81, XP028176327, ISSN: 1883-1958, DOI: 10.1016/J.JPOR.2010.09.003 [retrieved on 2010-09-27]

## Description

### Technical field

The present invention refers to the technical field concerning medical instruments.

In particular, the invention refers to a device allowing to detect, interrupt, monitor and treat parafunctional activities over time, in particular those regarding odontological field.

### Background art

The parafunctional activity is defined as an abnormal, not physiological, muscular activity without functional significance, which occurs between a function and another one, while instead the musculature should rest.

The parafunctional activity may occur both while awake and while asleep.

Almost the entirety of parafunctional patients does not realize their parafunctional activity but only suffers symptoms of damages caused by this disease. Among the various parafunctional activities in odontological field, the most important (as they are more damaging) and frequent ones are bruxism, teeth-grinding and jaw clenching. Under normal conditions, when the mouth does not move and therefore is at rest, mandibular posture is determined by the balance of the muscular tones and at this stage the teeth are not in contact with each other. Physiologically, the teeth of the antagonist arches come into contact with each other only, and not always, while swallowing; this means that, if the antagonist teeth contact each other outside of the swallowing, then a parafunctional activity is occurring. Therefore, the common denominator of these parafunctions is the dental contact outside swallowing. Depending on the type of contact and the mandibular movement, bruxism is distinguished from teeth-grinding and jaw clenching. Parafunctions occur both while asleep and while awake and the damage caused by this disease is directly proportional to their intensity (power) and frequency. The most frequent damages caused by the parafunctional activity concern:
- Teeth (teeth wear, thermal sensitivity, fractures, mobility);
- Gums (recessions, inflammations, gingival pockets);
- Bone (rarefaction, bony pockets);
- Muscles (pain near the ear, temple, under the cheekbone, the jaw, neck, back, during chewing, muscle-tense headache, limitation to open and move the mouth);
- Joints (joint noises near the ear, articulatory snap while opening and closing the mouth, arthritis and mandibular arthrosis);
- Vertigo;
- Tinnitus;
- Sleep disorders.

At the present time, many electronic devices exist trying to detect a parafunctional activity, in order to emit a signal or input persuading the patient to stop it.

For example, on 27/11/2015 the same inventor of the present PCT application filed an Italian patent application with number UB2015A005983, in which he describes a device that can be applied onto skin, for example behind the ear.

The device includes a sensor (S) able to detect muscular activity and a processor (P) programmed to recognize a normal muscular activity from a parafunctional activity, analyzing the duration time (T) of the detected muscular activity and the time interval between an activity and the subsequent one.

Therefore, in the case of parafunctional activity, it occurs generally according to specific parameters of frequency and duration different from physiological ones.

Other known similar devices work according to that principle, for example WO98/31277.

Further devices and methods for detecting parafunctional activity according to the state of the art are for instance described in:
DE202009012470U1,
US2006/184059
   M. FUJISAWA ET AL: "Determination of daytime clenching events in subjects with and without self-reported clenching", JOURNAL OF ORAL REHABILITATION, vol. 40, August 2013, pages 731-736, XP055476978,GB ISSN: 0305-182X, and
   AKIRA WATANABE ET AL: "Effect of electromyogram biofeedback on daytime clenching behavior in subjects with masticatory muscle pain" , JOURNAL OF PROSTHODONTIC RESEARCH, ELSEVIER, AMSTERDAM, NL, vol. 55, no. 2, 20 September 2010, pages 15-81, XP028176327, ISSN: 1883-1958.

A technical problem is that devices working only by means of an analysis of electromyographic signals, for example in terms of duration and frequency of muscular activity, are not perfectly accurate and functional, especially in evaluating a phenomenon during the waking period.

During the waking period, but also during sleep, although to a lesser extent, the facial mimic activity associated with speech is frequent and continues and its duration, frequency or intensity detected by sensors can lead to mix up such voluntary activity with a parafunctional activity.

While asleep, vocal activity is minimized (for example, it is possible to talk involuntarily if you are dreaming) but anyway this event may be mixed up with a parafunctional activity. During the day, this activity is so frequent and random that a correct interpretation of the parafunctional activity becomes very difficult.

### Summary of invention

It is therefore the aim of the present invention to provide an innovative device which solves said technical inconveniences.

In particular, the aim of the present invention is to provide a device for monitoring a parafunctional activity, in particular that related to a user's mouth (of odontological type), effectively and without causing any discomfort both while asleep and while awake.

More particularly, the object of the present invention is to provide a reliable device able to detect the parafunctional action even during the waking hours wherein the subject speaks, thus actuating the facial expression.

These and other aims are thus obtained through the present device (1) for detecting a parafunctional activity, according to claim 1.

Such a device (1) comprises sensing means (3, 103) configured to detect a muscular activity and a processor (110) programmed for recognizing a parafunctional activity by means of an analysis of the electromyographic signal detected by said sensing means.

According to the invention, a sound detecting device (150, C) is further comprised, cooperating with the processor in such a manner as to distinguish a potential vocal sound emitted in use by the user.

In this way, all said technical inconvenience are easily solved.

Indeed, the sound detecting device is able to detect the voice of the subject wearing the device and to send a related signal to the processor.

In particular, the sound detecting device, a microphone for example, can only transduce physically into signal all the surrounding sounds (voice, noise, etc.). Analogic band-pass filters being part of the sound detecting device limit this signal into a frequency range centered on the human voice. The processor, for example a microcontroller, acquires the filtered signal and then establishes with internal parameters (i.e. specific algorithm) whether the sound is compatible with the patient's voice.

In this way, in an event of contextual detection of an electromyographic signal received by the sensing means showing a parafunctional activity and of the recognition of a vocal sound emitted by the user, the processor categorizes such an event as a false positive.

Instead, in the case of detection of only an electromyographic signal proving a parafunctional activity without any detection (i.e. without any recognition) of a vocal sound, the processor identifies such an occurrence as parafunctional activity and can memorize the occurrence and possibly activate also the dissuading device (a vibration, for example).

In this way, in a secure manner, it is possible to identify reliably and discard all the false positives determined by the facial expression of the user while speaking.

It is also described here a method for detecting a parafunctional activity and comprising the detection of an electromyographic signal by means of sensing means (3, 103) configured to detect a muscular activity and an analysis of said signal through a processor (110).

According to such a method, a phase of vocal recognition of the subject is further comprised by means of a sound detecting device (150, C) cooperating with the processor in such a manner that in the event of concomitant detection of an electromyographic signal indicating a parafunctional activity and of recognition of the vocal sound emitted by the user, the processor categorizes such an occurrence as a false positive and in the case of detection of only the electromyographic signal indicating a parafunctional activity without the recognition of a vocal sound emitted by the user, the processor identifies such an occurrence as a parafunctional activity.

Further advantages are inferable by the other remaining dependent claims.

### Brief description of the drawings

Further features and advantages of the present device (1; 101), according to the invention, will become apparent from the following description of preferred embodiments thereof, given only by way of non-limiting, indicative example, with reference to the accompanying drawings, wherein:
- Figure 1 shows a schematization of the device 1, according to a first embodiment, which for example can be applied behind the earlobe and extended onwards to the tragus and downwards to the angle of the mandible, in such a manner as to detect the muscular activity of the masseter muscle;
- Figure 2 shows a time advancement of the muscular activity;
- Figure 3 shows a block diagram of the present invention;
- Figure 4 shows a wireless communication between the said device, object of the invention, and a device detecting such data, for example a mobile telephony device;
- Figure 5 shows a block diagram of the device 101 according to a second embodiment equal to the first except for the addition of a sound detecting device (C, 150) cooperating with the processor 110;
- Figure 6 refers to block A depicted in figure 5, wherein the circuitry required to amplify the signal read by the superficial electromyography sensors is depicted; the analogic signal acquired by differential method is amplified and filtered so that it can be digitally converted;
- Figure 7 shows block B depicted in figure 5 and particularizes a portion of the circuits, useful to rectify and integrate the signal. The depicted analogical one is useful for making the analysis and the analog/digital conversion of the signal easier;
- Figure 8 shows a block C always depicted in figure 5 and representing the filters for cleaning the signal received by the microphone; such figure 8 depicts the electrical scheme of one of the possible implementations useful to filter the sound acquired by the microphone; the depicted filter can be used to filter the frequencies of the human voice over other surrounding sounds.
- Figure 9 is a schematic layout showing the application of the option with the microphone close to the ear; the double arrows show the case of voice detection together with the detection of muscular activity due to speaking and therefore it is decoded as a false positive;
- Figure 10 is an overall flowchart highlighting the operating phases in accordance with this embodiment with microphone.

### Description of some preferred embodiments

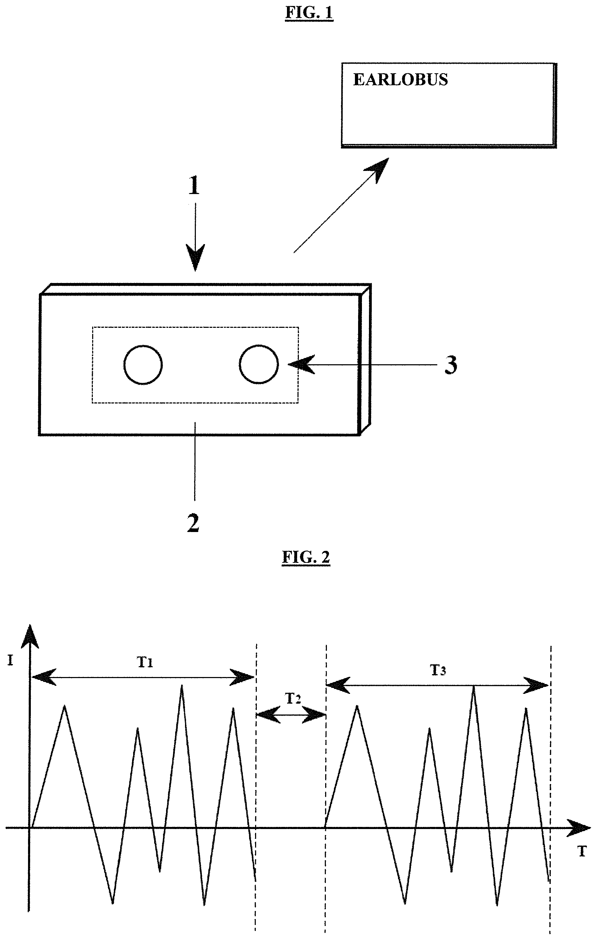

With reference to figure 1, according to a first embodiment of the invention, it is described here a device 1 which can be applied onto the skin, by means of, for example, a surface or an adhesive support.

The adhesive support 2 can be in the form of a sheet on which the further described elements are arranged. Therefore, it is similar to a normal patch.

The adhesive part can be preferably of the interchangeable type and, for example, providing an additional double-sided adhesive sheet connected to the sheet forming the support for the parts described further.

The overall sizes of the adhesive support can vary depending on needs but, thanks to the presently available technologies, very small sizes can be provided. Therefore, this allows an application onto the skin in suitable positions preventing the device from causing disturbances or inconveniences, thus proving not to be invasive but functional.

Moreover, as it will be described below, all the components are integrated in said adhesive support, such that no physical connections to external PCs are needed by means of wirings in general, making such a device very convenient when using it both while sleeping and while being awake.

Thanks to the adhesive support, such a device can be placed by the patient onto the skin either behind, or ahead or just below the earlobe, in correspondence of the angle of the mandible and near the skin part of the mandibular insertion of the masseter muscle or, alternatively, it can be always placed onto the skin ahead and just above the ear by the skin part of the mandibular insertion of the temporal muscle. These positions are convenient, as the sensor can work by detecting the muscular activity of said two muscles, the most used ones during the mouth parafunctional activity.

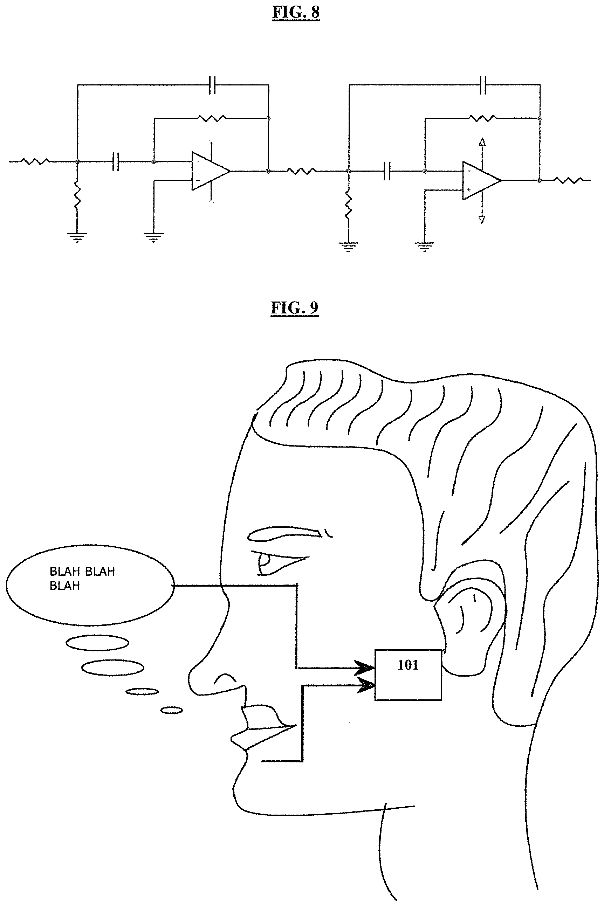

Figure 9 depicts schematically an illustrative example of application, even if showing the specific case of the second embodiment described below.

Therefore, figure 1 outlines the adhesive support 2 and highlights a further area 3.

Such an area 3 includes a system of sensors able to detect muscular activity, for example two sensors of superficial electromyography.

In particular, as commonly used in the field of electromyography, two sensors are preferably provided, i.e. a couple of electrodes. A third auxiliary sensor, or electrode, is generally used for reducing the noise.

Each sensor can be for example of the type of an electrode or an electrode array able to detect the electric (electromyographic) signals produced by the contraction of the local musculature affected by the parafunctional activity. Therefore, the device 1 is generally applied to a point on the skin and the detecting system 3 is able to read the corresponding muscular activity in the area where it is applied.

Such sensors are already known per se in the background and they are used in the field of electromyography, also for different purposes and uses, and for this reason they are not structurally described further here.

Alternatively, sensors made by a deformable electric element which modifies its resistance to the electrical current passage according to its shape could be used. Its deformation indicates muscular activity and it can be measured by checking its resistance variation with respect to a standard value determined by its rest condition.

Then a system for ADC conversion is provided integrated in the adhesive support. Indeed, the sensor provides analogic data which must be converted into digital for their subsequent memorization, sending and processing.

Then a buffer/processor memory is provided. Such a processor analyses data sent by the sensor unit and its task is to recognize voluntary and physiological muscular activity (for example swallowing) from the parafunctional activity, as it is better described further.

An actuating device for correcting the parafunctional activity is always integrated in such an adhesive support. For this purpose, such a device activates a response signal in the case of a parafunctional activity, for example a short vibration in this case (produced for example by a piezoelectric actuator or a similar one, as for the "vibration" function of a common cellphone). In another embodiment, such an actuating device can inject a short electrical current with such a frequency not to cause pain or ache to the patient.

Then a memory card is provided for memorizing data related to the parafunctional activity.

An antenna allows to transmit memorized data to an external device, in particular to an application (for example on the patient's personal cellphone) which on its turn transmits them to a detecting station.

Last a battery, held in the device itself, is provided for supplying energy to the system and for executing the functions of sensing, vibration, memorization and sending of wireless data.

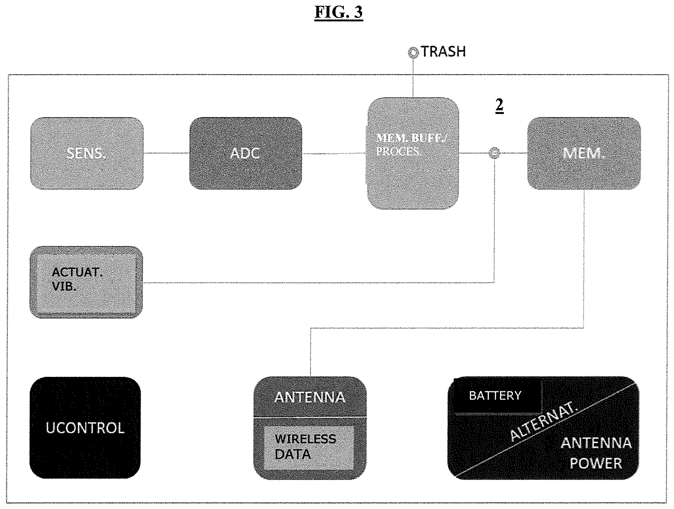

A scheme which shows de *facto* such components arranged on the adhesive support is depicted in figure 3.

As shown in figure 2, the principle for determining an odontological parafunctional activity through the sensing system is based on the determination of electromyographic signals detected by the sensor which prove a parafunctional activity.

In a preferred embodiment of the invention such electromyographic signals can be the duration time (T) of the muscular activity and its frequency, wherein frequency means here the time interval between an occurrence and the following one.

Indeed, swallowing occurs by activating the musculature for a duration of a second approximately and with a frequency of no more than once a minute while, in the case of parafunctional activity, the musculature is activated for more than a second (i.e. the duration time of muscular activity is more than a second) and with a frequency of less than a minute (i.e. the rest interval between an occurrence and the subsequent one is less than a minute).

Accordingly, the processor receiving the signals of muscular activity can be easily programmed on such bases (i.e. duration and frequency) in such a manner as to recognize a parafunctional activity from a normal function, which is then rejected and not recorded. Therefore, the variables under monitoring are the duration of the muscular activity (T) and the time interval (ΔT) when the muscular activity stops (i.e. frequency). What is considered as a function is rejected while the parafunctional activity is recorded on a memory, as per the scheme of figure 3, and allows the activation of the actuator (for example, with a vibration) for the response.

Therefore, if the detected muscular activity is comprised in predetermined ranges of duration and frequency, this is categorized as a parafunctional activity.

A further memory is designated to send data to an external device by means of an antenna, as described above.

Therefore, only for example, figure 2 shows a first occurrence detected by the sensor, measuring a series of electric pulses during time T1. This shows the fact that muscle activity, independently of the applied muscular power, is recorded for a certain time T1. Subsequently, the sensor records a pause interval T2 and then begins to record a new T3 duration phenomenon.

As a matter of principle, the indicative ranges of a parafunctional activity are those for which each event has a duration longer than one second: T1> 1 sec; and the interval between an occurrence and the next one less than one minute: ΔT <1 min.

Therefore, the processor is programmed to evaluate these time periods of muscular activity and subsequent pauses between an activity and the next one. If a T1 activity and a T3 activity are detected in a given time interval above a predetermined threshold value with a T2 pause below a predetermined threshold value, then as above described, this is recognized as a parafunctional phenomenon which is stored and the actuator is activated at the same time.

The vibration is preferably at 120Hz since such frequency is known to be used for stretch musculature and therefore it is suitable for such purposes.

In a further embodiment of the invention, such electromyographic signals that are analyzed for the determination of a parafunctional activity concern the duration of the occurrence and the muscular intensity (I).

Therefore, if the duration and muscle intensity fall within predetermined values showing parafunctional activity (programmed values and known to the processor exactly as in the case above), the processor identifies this event as a parafunctional activity.

The processor could be programmed to detect parafunctional activity by analyzing only one or a combination of said parameters among duration, frequency and intensity of muscular activity.

Obviously, any electromyographic signal indicative or useful for determining a parafunctional activity can be generally used.

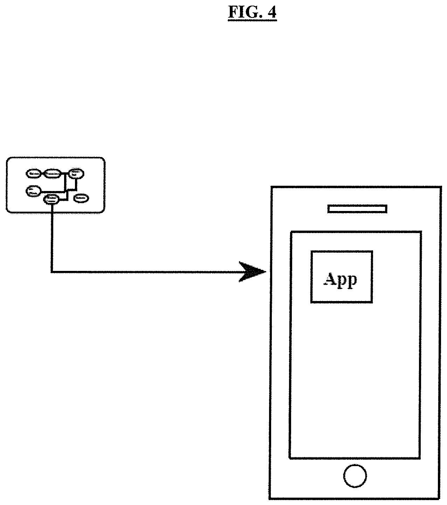

As described, the system can easily include, as shown in figure 4, wireless communication with an external device, for example through an application APP on the patient's cellphone and which, in turn, allows to store data.

In this way, the user normally logs into the application and the system uploads such data onto the application.

Then a medical center may have access to such data to monitor patients.

As mentioned, all the components are of small size, such as a coin, and can be applied to an adhesive support. However, the support can also not be adhesive and can connect with different support systems, such as a small belt to tie around the neck.

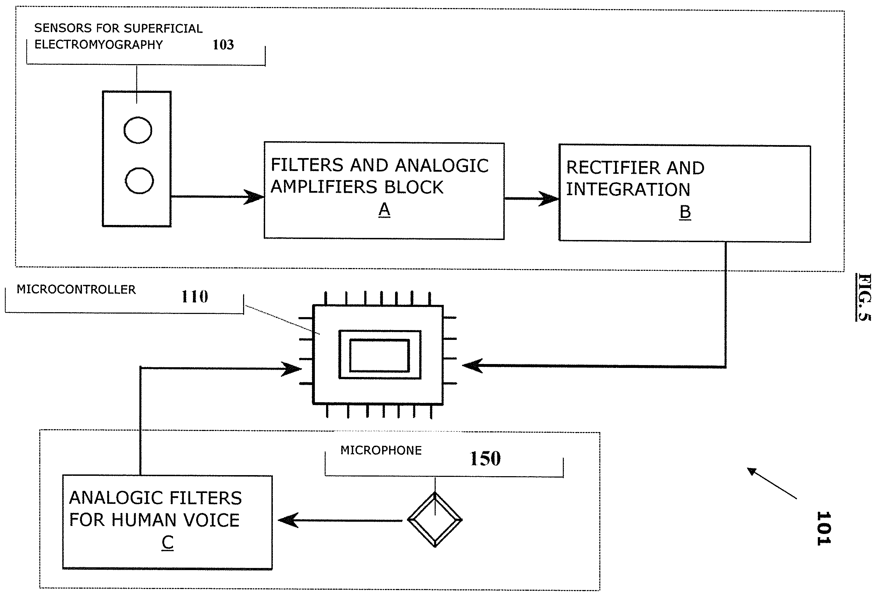

Subject to what has been described above, a preferred embodiment of the invention is described with reference to subsequent figures 5 to 10.

To this variant applies everything described above, in particular in terms of electromyographic detection to detect indicative parafunctional muscular activity as well as the provided components.

Therefore, in this case, subject to what has been described above, the new embodiment adds a microphone 150 in order to exceed predetermined functional limits due to "false positives" determined by vocal activity.

As previously introduced in the field of technical inconveniences, it is known that vocal activity (i.e. speaking) may cause an error (a false positive), as this activity determines muscular activity detected by electromyographic sensors which may be included in the parameters identifying a parafunctional activity. Depending on the duration of the vocal action, the muscular activity linked to it may likely be included in said parameters of duration, frequency and/or muscular intensity, with a real risk of being mixed up with parafunctional activity, as it is actually a false positive.

Indeed, above all during daytime activities and waking hours, the subject may speak and therefore have such a muscular activity that could easily fall within a frequency and/or duration and/or intensity range related to the parafunctional activity.

Indeed, during the speech, the sensors detect muscular activity, which obviously must be rejected.

The insertion of the microphone, as explained below, solves this technical inconvenience by allowing it to check whether voice recognition is associated with the detection of muscular activity at the same time.

Figure 5 shows an overall outline according to this solution.

As previously described, the whole can be arranged on a support, for example adhesive, applicable for example near the ear lobe, as shown in the schematization of figure 1, or on a support of a different type.

In this embodiment, there are arranged on the support:
The processor 110 represented in the form of a microcontroller and obviously also present in the first embodiment. The processor manages the various operations and recognizes whether the received information can be classified as a parafunctional activity or not exactly as in the previously described embodiments and therefore according to the above-mentioned modalities of muscular frequency and/or muscular intensity and/or duration of the muscular occurrence.

As explained in the following, the microcontroller adds to this function a function of voice recognition that includes the use of a voice recognition algorithm based on a possible signal received by the microphone.

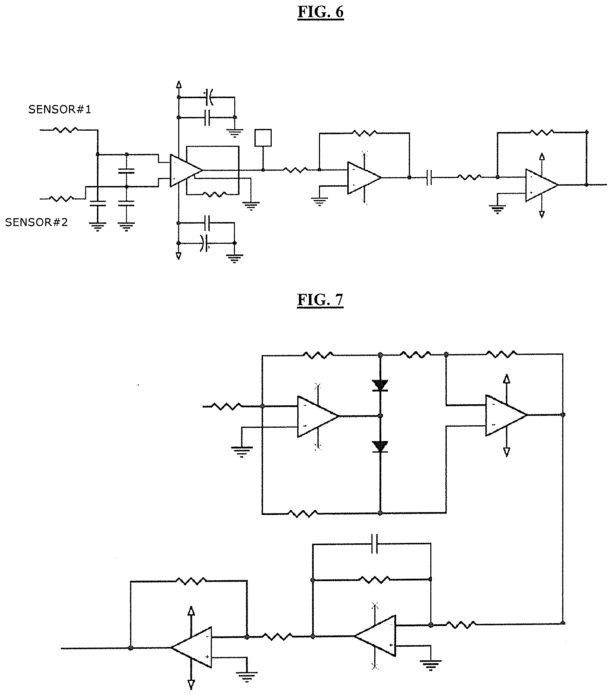

Figure 5 shows two sensors of electromyographic surface 103 to which the electronic components are connected, represented by blocks (A) and (B) of figures 6 and 7, being also present on the first embodiment. They refer in fact to various filters and rectifiers which, as per background art, make the signal clean and readable to the microcontroller.

According to the schematization of figure 5, the microphone 150 is then provided for acquiring sounds emitted in the surroundings.

In the preferred embodiment of the invention, the microphone is integrated in the support itself and then its suitable size is like a pinhead.

Nowadays many suitable types of microphones with such sizes exist. The chosen useful technology has the purpose of an easy insertion of the microphone into the device both for reducing its sizes and encumbrance and for guaranteeing at the same time enough sound perception to easily detect the patient's voice.

For that purpose, the device includes preferably a microphone of the MEMS type or other next technology useful for allowing both small sizes and enough sound perception useful to detect the sound deriving from the patient's voice.

A possible embodiment of the invention including a throat microphone is not excluded.

Obviously, comprising the integration of microphone in the same adhesive support allows a much smaller size solution.

The filters (block C of figure 5) arranged downstream the microphone allow to cancel background noises.

Such filters are described in detail in figure 8 and are shown as block C in figure 5. They are able to remove the background noise and so to transmit only the user's voice to the microprocessor, if the voice is emitted.

In particular, the embodiment is as follows: MICROPHONE - FILTERS/AMPLIFICATION - MICROPROCESSOR. The microphone is physically sensitive to every noise. Then filters and amplifiers "clean" the signal from most part of the background noise. Then the microprocessor, with suitably arranged internal algorithms, analyzes the signal to decide whether the received signal is compatible with the sound emitted by the patient.

Therefore, if no voice is emitted by the user but there is background noise, such filter C does not transmit any useful information to the microcontroller or anyway the received information is then rejected by the processor thanks to its algorithm of recognition of the patient's voice.

The filter, as per the scheme of figure 8, is based on band-pass filters focused on the distinctive frequency interval of the human voice.

Obviously, with reference to figure 5, the dissuading device and the potential system for communicating with the external software can be also included in the support, exactly as described in the previous embodiments.

The device comprises, at the start, an initial calibration phase to modulate levels depending on the signal/noise ratio of the contact and to evaluate the subjective signal level of the patient.

Basically, an initial calibration by emitting a vocal sound by the user determines an indicative reference. The device is based not only on the information acquired during the calibration process but also compares the signal with internal models to categorize whether muscular activity is comparable or not to that generated by a parafunctional activity.

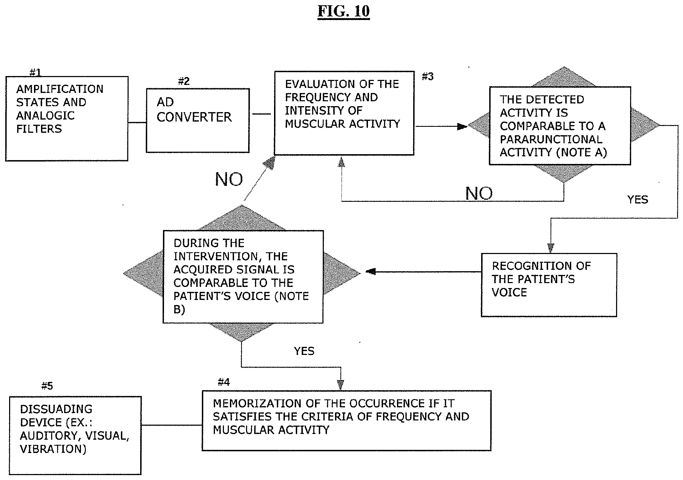

At this point, as shown by the flowchart of figure 10, the operation is as follows:
When there is muscle activity, the sensor (or the sensors) detects that activity which is sent to microcontroller 110, as shown in diagram of figure 5.

The microcontroller must check whether this muscular activity falls or not into a parafunctional activity case by verifying, with reference to such muscular activity, frequency and/or duration and/or muscular intensity. If detected frequency and/or duration and/or muscular intensity, as mentioned above, fall into predetermined preset ranges, then this is potentially recognized as a parafunctional activity.

This is shown in the first part of the flowchart marked with the steps (#1, #2 and #3) until arriving to the verification phase if the detected activity is comparable to a parafunctional activity. If it is not, a new muscle activity has to be analyzed and therefore the micro-controller does not carry out any operation.

On the contrary, in the case that the electromyographic signals detected through the sensors are recognized by the microcontroller as a possible parafunctional event, then the analysis of the possible signal received by the microphone occurs. In this case, the direction of the arrow indicated by YES in the flowchart leads to the voice recognition ("Patient's Voice Recognition" box).

In that sense as already said the microphone enters in function because, if the user's voice has been detected together with muscular activity, then most probably it is not a parafunctional activity but it is only "speaking".

The provided filters, as mentioned, cancel background noise, so if the microphone detects a sound, this is most probably the voice of the patient.

The processor analyzes the filtered signal coming from the microphone with a special algorithm to categorize it as a voice or not.

Therefore, if this sound is categorized as the emission of patient's vocal sound, then the microprocessor simultaneously receives both an electromyographic signal from the sensors compatible with a parafunctional activity and a signal by the filters downstream the microphone, compatible as voice, it classifies this information as a false positive. Indeed, the possible signal of parafunctional activity is linked to a muscular activity of speaking.

However, if in correspondence of the electromyographic signal indicating a parafunctional activity, the microcontroller does not receive any useful signal by the microphone or receives a signal categorized as a background noise, then this information is categorized as a parafunctional activity.

Therefore, if the microcontroller does not receive any signal, filtered by the above-mentioned filters, by the microphone that is compatible with the patient's voice then the detected activity is classified as a parafunctional activity, if it falls within the preset frequency and duration parameters.

Then this occurrence is stored with the subsequent actuation of the dissuading device.

Otherwise, if the microcontroller receives a signal, filtered by the above-described filters, from the microphone that is compatible with the voice of the patient, and at the same time the activity received by the sensors falls among the parafunctional activities, then this event is rejected as a false positive.

Ultimately, always with reference to the flowchart of figure 10:
Sensors to check muscular activity are always operating.

When the device measures a muscular activity over certain criteria (such as frequency and intensity as mentioned), then the device enters the "DETECTION" mode (interval of a few seconds).

Under this mode, in addition to muscular activity, the microphone signal is also acquired.

In this "DETECTION" range, it is assessed whether the activity is comparable to a parafunctional activity or not. In particular, if a micro-controller receives a sound signal coming from the microphone and its filters, then this is analyzed and if it is indicating the voice, referring to the flowchart of figure 10, the NO line is followed, leading to wait for a new occurrence.

On the contrary, if no voice is detected, then the YES line that leads to the storing and the implementation of the dissuading device is followed.

The microcontroller, therefore, does not always analyze the voice (or more generally the surrounding sound), but analyzes and acquires the voice only when necessary.

The electromyographic signals for detecting a parafunctional activity are those cited in the description and any other known ones used for a long time to verify a parafunctional activity and they are not a specific object of the present invention. For this reason, they are not described here further.

Finally, in a further embodiment of the invention, subject to what has been described above, the present device can be associated with a further stress detection device, for example in the form of a wearable bracelet.

The bracelet, similarly to the described device, can include sensors suitable for detecting body stress, together with the same filters and signal A/D converters. Both devices can be communicating with a data recording device, such as the App. provided in the mobile telephony device. In this manner, once stored, a whole monitoring can be examined by specialized personnel. Such body stress indicators related with the detected occurrences of parafunctional activities can be useful in determining a correct case history of the subject and for example identifying a correct therapy, as well as providing statistical correlation data between parafunctional activity and stress states.

The invention is defined in the appended claims.

## Claims

1. A device (1) for detecting a parafunctional activity and comprising:
- Sensing means (3, 103) configured to detect an electromyographic signal produced by a muscular activity;
- A processor (110) programmed for recognizing a parafunctional activity by means of an analysis of the said electromyographic signal;
- A sound detecting device (150, C), cooperating with the processor (110) in such a manner as to distinguish a potential vocal sound emitted in use by the user;
- A dissuading device for interrupting the parafunctional activity;
- wherein the processor and the sound detecting device cooperate with each other in such a manner that, in the event of concomitant detection of an electromyographic signal compatible with a parafunctional activity and of recognition of the vocal sound emitted by the user, the processor categorizes such an occurrence as a false positive and in the case of detection of the electromyographic signal compatible with a parafunctional activity without the recognition of a vocal sound emitted by the user, the processor identifies such an occurrence as a parafunctional activity, said device being provided with an adhesive support, able to be applied to the user's face, on which said sensing means, the processor and the sound detecting device are arranged and wherein, further, said dissuading device is integrated in said adhesive support and is activated in response to the identification of said parafunctional activity in the said case of detection of the electromyographic signal compatible with a parafunctional activity without the recognition of a vocal sound emitted by the user.

2. A device (1), according to claim 1, wherein the sound detecting device comprises at choice:
- A microphone;
- A throat microphone.

3. A device (1), according to claim 2, wherein the microphone is of the MEMS type.

4. A device (1), according to one or more of the preceding claims, wherein the sound detecting device further comprises an analogic filter (C) for the human voice, preferably an analogic band-pass filter.

5. A device (1), according to one or more of the preceding claims, wherein the processor is a microcontroller.

6. A device (1), according to one or more of the preceding claims, wherein a block (A) of analogical filters and amplification and a block (B) of rectifier and integration are further comprised for the signals coming from the sensor upstream the processor.

7. A device (1), according to one or more of the preceding claims, wherein said sensing means (3, 103) are in the form of a sensor of surface electromyography.

8. A device (1), according to one or more of the preceding claims, wherein two or more than two sensors of surface electromyography are provided.

9. A device (1), according to one or more of the preceding claims, wherein the processor is programmed to check if an electromyographic signal is indicating a parafunctional activity on the basis of the frequency between an occurrence of a signal and the subsequent one and the duration of each signal.

10. A device (1), according to one or more of the preceding claims, wherein the processor is programmed to check if an electromyographic signal is indicating a parafunctional activity on the basis of the frequency between an occurrence of a signal and the subsequent one and the intensity of the signal.

11. A device (1), according to one or more of the preceding claims, wherein the processor is programmed to check if an electromyographic signal is indicating a parafunctional activity on the basis of the analysis of at least one of the following elements:
- Frequency between an occurrence of a signal and the subsequent one;
- Intensity of the signal;
- Duration of each signal.

12. A device (1), according to claim 1, wherein said dissuading device is a vibrating device able to cause a vibration in the subject.

13. An assembly comprising a device, as per one or more of the preceding claims, and a second device comprising one or more sensors for detecting the status of body stress.

14. The device, according to one or more of the previous claims from 1 to 12, configured for treatment of a parafunctional activity.

## Patentansprüche

1. Eine Vorrichtung (1) zur Erfassung einer parafunktionellen Aktivität, die umfasst:
- Sensormittel (3, 103), die so konfiguriert sind, dass sie ein elektromyographisches Signal erfassen, das durch eine Muskelaktivität erzeugt wird;
- ein Prozessor (110), der so programmiert ist, dass er eine parafunktionelle Aktivität mittels einer Analyse des elektromyographischen Signals erkennt;
- eine Geräuscherkennungsvorrichtung (150, C), die mit dem Prozessor (110) so zusammenarbeitet, dass sie einen potenziellen Stimmschall, der beim Gebrauch vom Benutzer abgegeben wird, unterscheiden kann;
- eine Abschreckvorrichtung zur Unterbrechung der parafunktionellen Aktivität;
- wobei der Prozessor und die Geräuscherkennungsvorrichtung derart miteinander zusammenarbeiten, dass im Falle der gleichzeitigen Erfassung eines mit einer parafunktionellen Aktivität kompatiblen elektromyographischen Signals und der Erkennung des vom Benutzer abgegebenen Stimmgeräusches der Prozessor ein solches Ereignis als falsch positiv einstuft und im Falle der Erfassung des mit einer parafunktionellen Aktivität kompatiblen elektromyographischen Signals ohne die Erkennung eines vom Benutzer abgegebenen Stimmgeräuschs der Prozessor ein solches Ereignis als parafunktionelle Aktivität identifiziert, wobei die genannte Vorrichtung mit einem klebenden Träger versehen ist, der auf dem Gesicht des Benutzers angebracht werden kann, auf dem die genannten Sensormittel, der Prozessor und die Geräuscherkennungsvorrichtung angeordnet sind, und wobei außerdem die genannte Abschreckvorrichtung in den genannten klebenden Träger integriert ist und als Reaktion auf die Identifizierung der parafunktionellen Aktivität im genannten Fall der Erfassung des mit einer parafunktionellen Aktivität kompatiblen elektromyographischen Signals ohne die Erkennung eines vom Benutzer abgegebenen Stimmgeräusches aktiviert wird.

2. Eine Vorrichtung (1) gemäß Anspruch 1, wobei die Geräuscherkennungsvorrichtung wahlweise umfasst:
- ein Mikrofon;
- ein Kehlkopfmikrofon.

3. Eine Vorrichtung (1) gemäß Anspruch 2, wobei das Mikrofon vom MEMS-Typ ist.

4. Eine Vorrichtung (1) gemäß einem oder mehreren der vorhergehenden Ansprüche, wobei die Geräuscherkennungsvorrichtung ferner einen analogen Filter (C) für die menschliche Stimme, vorzugsweise einen analogen Bandpassfilter, umfasst.

5. Eine Vorrichtung (1) gemäß einem oder mehreren der vorangehenden Ansprüche, wobei der Prozessor ein Mikrocontroller ist.

6. Vorrichtung (1) gemäß einem oder mehreren der vorhergehenden Ansprüche, wobei ferner für die vom Sensor stromaufwärts des Prozessors kommenden Signale ein Block (A) aus analogen Filtern und Verstärkung und ein Block (B) aus Gleichrichter und Integration enthalten sind.

7. Eine Vorrichtung (1) gemäß einem oder mehreren der vorhergehenden Ansprüche, wobei die Sensormittel (3, 103) in Form eines Sensors für Oberflächen-Elektromyographie vorliegen.

8. Eine Vorrichtung (1) gemäß einem oder mehreren der vorhergehenden Ansprüche, wobei zwei oder mehr als zwei Sensoren für die Oberflächen-Elektromyographie vorgesehen sind.

9. Eine Vorrichtung (1) gemäß einem oder mehreren der vorhergehenden Ansprüche, wobei der Prozessor so programmiert ist, dass er anhand der Häufigkeit zwischen dem Auftreten eines Signals und dem darauffolgenden und der Dauer jedes Signals prüft, ob ein elektromyographisches Signal eine parafunktionelle Aktivität anzeigt.

10. Eine Vorrichtung (1) gemäß einem oder mehreren der vorhergehenden Ansprüche, wobei der Prozessor so programmiert ist, dass er anhand der Häufigkeit zwischen dem Auftreten eines Signals und dem darauffolgenden und der Intensität des Signals prüft, ob ein elektromyographisches Signal eine parafunktionelle Aktivität anzeigt.

11. Eine Vorrichtung (1) gemäß einem oder mehreren der vorhergehenden Ansprüche, wobei der Prozessor so programmiert ist, dass er prüft, ob ein elektromyographisches Signal eine parafunktionelle Aktivität anzeigt, und zwar auf der Grundlage der Analyse von mindestens einem der folgenden Elemente:
- Häufigkeit zwischen dem Auftreten eines Signals und dem darauffolgenden;
- Intensität des Signals;
- Dauer jedes Signals.

12. Eine Vorrichtung (1) gemäß Anspruch 1, wobei die Abschreckvorrichtung eine vibrierende Vorrichtung ist, die in der Lage ist, eine Vibration in dem Subjekt zu verursachen.

13. Eine Baugruppe, die eine Vorrichtung gemäß einem oder mehreren der vorhergehenden Ansprüche umfasst und eine zweite Vorrichtung, die einen oder mehrere Sensoren zur Erfassung des Status der Körperbelastung enthält.

14. Eine Vorrichtung gemäß einem oder mehreren der vorhergehenden Ansprüche 1 bis 12, die zur Verarbeitung einer parafunktionellen Aktivität konfiguriert ist.

## Revendications

1. Dispositif (1) de détection d'une activité parafonctionnelle et comprenant°:
- des moyens de détection (3, 103) configurés pour détecter un signal électromyographique produit par une activité musculaire°;
- un processeur (110) programmé pour reconnaître une activité parafonctionnelle au moyen d'une analyse dudit signal électromyographique°;
- un dispositif de détection de sons (150, C), coopérant avec le processeur (110) de manière à distinguer un potentiel son vocal émis à l'usage de l'utilisateur°;
- un dispositif dissuasif pour interrompre l'activité parafonctionnelle°;
- dans lequel le processeur et le dispositif de détection de sons coopèrent l'un avec l'autre de telle manière que, en cas de détection concomitante d'un signal électromyographique compatible avec une activité parafonctionnelle et de reconnaissance du son vocal émis par l'utilisateur, le processeur classe un tel événement comme faux positif et en cas de détection du signal électromyographique compatible avec une activité parafonctionnelle sans la reconnaissance d'un son vocal émis par l'utilisateur, le processeur identifie un tel événement comme activité parafonctionnelle, ledit dispositif étant pourvu d'un support adhésif, apte à être appliqué sur le visage de l'utilisateur, sur lequel sont disposés lesdits moyens de détection, le processeur et le dispositif de détection de sons et dans lequel, en outre, ledit dispositif dissuasif est intégré dans ledit support adhésif et est activé en réponse à l'identification de ladite activité parafonctionnelle dans ledit cas de détection du signal électromyographique compatible avec une activité parafonctionnelle sans la reconnaissance d'un son vocal émis par l'utilisateur.

2. Dispositif (1) selon la revendication 1, dans lequel le dispositif de détection de sons comprend au choix°:
- un microphone°;
- un microphone pour la gorge.

3. Dispositif (1) selon la revendication 2, dans lequel le microphone est du type MEMS.

4. Dispositif (1) selon une ou plusieurs des revendications précédentes, dans lequel le dispositif de détection de sons comprend en outre un filtre analogique (C) pour la voix humaine, de préférence un filtre passe-bande analogique.

5. Dispositif (1) selon une ou plusieurs des revendications précédentes, dans lequel le processeur est un micro-contrôleur.

6. Dispositif (1) selon une ou plusieurs des revendications précédentes, dans lequel un bloc (A) de filtres analogiques et d'amplification et un bloc (B) redresseur et d'intégration sont en outre compris pour les signaux provenant du capteur en amont du processeur.

7. Dispositif (1) selon une ou plusieurs des revendications précédentes, dans lequel lesdits moyens de détection (3, 103) se présentent sous la forme d'un capteur d'électromyographie de surface.

8. Dispositif (1) selon une ou plusieurs des revendications précédentes, dans lequel deux ou plus de deux capteurs d'électromyographie de surface sont prévus.

9. Dispositif (1) selon une ou plusieurs des revendications précédentes, dans lequel le processeur est programmé pour vérifier si un signal électromyographique indique une activité parafonctionnelle sur la base de la fréquence entre une occurrence d'un signal et la suivante ainsi que de la durée de chaque signal.

10. Dispositif (1) selon une ou plusieurs des revendications précédentes, dans lequel le processeur est programmé pour vérifier si un signal électromyographique indique une activité parafonctionnelle sur la base de la fréquence entre l'occurrence d'un signal et la suivante ainsi que de l'intensité du signal.

11. Dispositif (1) selon une ou plusieurs des revendications précédentes, dans lequel le processeur est programmé pour vérifier si un signal électromyographique indique une activité parafonctionnelle sur la base de l'analyse d'au moins l'un des éléments suivants :
- fréquence entre une occurrence d'un signal et la suivante°;
- intensité du signal°;
- durée de chaque signal.

12. Dispositif (1) selon la revendication 1, dans lequel ledit dispositif dissuasif est un dispositif vibrant capable de provoquer une vibration chez le sujet.

13. Ensemble comprenant un dispositif, selon une ou plusieurs des revendications précédentes, et un second dispositif comprenant un ou plusieurs capteurs pour détecter l'état de stress corporel.

14. Dispositif, selon une ou plusieurs des revendications précédentes 1 à 12, configuré pour le traitement d'une activité parafonctionnelle.
